## Europäisches Patentamt
## European Patent Office
## Office européen des brevets

(11) Numéro de publication: **0 233 429 B1**

# (12) FASCICULE DE BREVET EUROPEEN

(45) Date de publication de fascicule du brevet: **29.09.93**

(51) Int. Cl.5: **A61K 47/00**, A61K 37/12,
//(A61K37/12,31:155)

(21) Numéro de dépôt: **86402920.2**

(22) Date de dépôt: **23.12.86**

---

(54) **Composition pharmaceutique contenant une association de collagène et d'un principe actif antiseptique et/ou anti-inflammatoire et son procédé de fabrication.**

---

(30) Priorité: **23.12.85 FR 8519035**

(43) Date de publication de la demande:
**26.08.87 Bulletin 87/35**

(45) Mention de la délivrance du brevet:
**29.09.93 Bulletin 93/39**

(84) Etats contractants désignés:
**AT BE CH DE ES FR GB GR IT LI LU NL SE**

(56) Documents cités:
**GB-A- 2 131 293**
**US-A- 3 435 117**
**US-A- 3 821 371**

**Pharmacopée européenne 2 Ed. 1981- Monographie 132.3**

**A. Le Mir "Abregée pharmacie galénique" 5 Ed.**

(73) Titulaire: **PIERRE FABRE MEDICAMENT**
**45, Place Abel Gance**
**F-92100 Boulogne(FR)**

(72) Inventeur: **Thomas-Leurquin, Geneviève**
**Faculté de Pharmacie de Lille-Pharmacotechnie**
**F-59045 Lille Cedex(FR)**
Inventeur: **Gayot, Anne**
**Faculté de Pharmacie de Lille-Pharmacotechnie**
**F-59045 Lille Cedex(FR)**
Inventeur: **Poitou, Pierre**
**5, Allée J.J. Bertrand**
**F-81100 Castres(FR)**
Inventeur: **Basquin, Serge**
**14, rue Borrel**
**F-81100 Castres(FR)**

(74) Mandataire: **Ahner, Francis et al**
**CABINET REGIMBEAU, 26, avenue Kléber**
**F-75116 Paris (FR)**

---

EP 0 233 429 B1

**Description**

La présente invention concerne une nouvelle composition thérapeutique solide consistant en une association entre du collagène et un principe actif antiseptique et/ou anti-inflammatoire, destinée au traitement des maladies parodontales, en particulier au traitement des poches parodontales profondes ou superficielles.

Le parodonte est l'ensemble des tissus de soutien de la dent. Il est formé par la gencive, et dans sa partie profonde par l'os alvéolaire, le cément et le desmodonte. Ce dernier est un ensemble de ligaments alvéolodentaires qui maintiennent fermement la dent dans le site alvéolaire.

Au niveau de la jonction dent-gencive, il existe un espace d'une profondeur d'environ 1 mm ; le sillon gingivo-dentaire qui est fermé par l'attache épithélio-conjonctive. Cette attache est une véritable barrière entre le milieu extérieur et le milieu interne.

La poche parondontale, conséquence de parodontites, peut être décrite comme le résultat d'un accroissement anormal de la profondeur du sillon gingivo-dentaire avec migration apicale de l'attache épithélio-conjonctive, par suite de réactions inflammatoires successives qui conduisent à la dégénération des tissus conjonctifs constitutifs des structures parodontales profondes.

Il est reconnu que le facteur étiologique principal de la maladie parodontale est d'origine bactérienne liée à la plaque dentaire.

L'absence de pratique d'hygiène bucco-dentaire se traduit par une accumulation de la plaque à la jonction dent-gencive et dans les espaces interdentaires. L'augmentation en volume de la plaque conduit à une gingivite qui évolue le plus souvent vers la parodontite.

L'inflammation progresse le long de la racine. Le passage de la gingivite à la parodontite est accompagné d'un changement dans la constitution de la flore bactérienne de la plaque dentaire.

Le traitement des poches parodontales consiste à éliminer les irritants locaux dont la plaque dentaire sousgingivale et à restaurer l'architecture osseuse et gingivale pour rendre le parodonte à nouveau fonctionnel.

L'apport d'un principe actif antibactérien dans la poche parodontale permet de contrôler efficacement le développement de la plaque dentaire sous-gingivale. Les moyens utilisés sont dans le cas de poches parodontales plus ou moins superficielles :

- l'irrigation quotidienne des poches parodontales avec une solution du principe actif, et
- l'introduction dans la poche parodontale de fibre creuse ou de film polymère contenant le principe actif et assurant dans la poche une libération prolongée du principe actif (quelques heures à quelques jours).

Ces moyens s'ils sont efficaces sur le plan bactériologique et clinique présentent cependant les inconvénients suivants :

- les irrigations impliquent la dextérité et l'observance du patient
- les films polymères, les fibres creuses ne sont pas résorbables et doivent être retirés de la poche après libération du principe actif, ce qui risque de provoquer un traumatisme des tissus.

Dans le cas d'affections plus graves, la restauration du parodonte fait appel le plus souvent à des techniques chirurgicales qui éliminent les tissus défectueux et repositionnent les tissus restants.

Dans certains cas des matériaux de comblement sont introduits dans les lésions infra-osseuses. Le choix des matériaux est basé sur l'induction éventuelle par ces matériaux d'ostéogénèse, de régénération tissulaire.

Ces produits présentent l'inconvénient de leurs préparation, conservation et manipulation.

Ainsi, il n'existe pas, à l'heure actuelle, de composition susceptible de traiter soit les poches parodontales superficielles sans provoquer de traumatisme des tissus, soit les poches parodontales plus profondes nécessitant en outre une intervention chirurgicale.

On sait par ailleurs que les ligaments alvéolodentaires, l'attache épithélio-conjonctive et, de façon plus générale, l'ensemble des tissus composant le parodonte, sont constitués en grande partie de collagène.

Le collagène est une glycoprotéine, représentant environ 40 % des protéines d'un organisme adulte. Le collagène, du fait de sa structure en triple hélice, et du fait des nombreuses liaisons hydrogène et de Van der Waals reliant entre eux les acides aminés le composant, est très stable et confère donc aux tissus conjonctifs des propriétés mécaniques remarquables. Il intervient, de plus, dans le processus de cicatrisation, d'une part en favorisant l'hémostase puisqu'il induit l'adhésion et l'agrégation plaquettaire, d'autre part en participant à l'élaboration d'un tissu de granulation dans lequel des cellules comme les fibroblastes viennent se développer et permettent ainsi la régénération des tissus. Et, comme la majorité des substances qui composent l'organisme, le collagène est soumis à un renouvellement permanent.

Le métabolisme du collagène varie avec l'âge, et d'un tissu à un autre. Au niveau des ligaments parodontaux, il est de 5 jours, c'est-à-dire 15 fois plus rapide qu'au niveau du derme.

De plus, du collagène exogène (par exemple, du collagène d'origine bovine) apporté dans l'organisme rejoindra le métabolisme du collagène endogène. Ce matériau est donc parfaitement résorbable. Par exemple, le temps moyen de résorption d'un collagène natif non dénaturé utilisé comme hémostatique est de 3 semaines. Le temps de résorption du collagène peut être augmenté par réticulation supplémentaire du collagène.

Par ailleurs, en comparaison avec d'autres protéines, le collagène est faiblement immunogène et, par exemple, parmi les déterminants antigéniques portés par le collagène humain, certains sont superposables à 95% avec ceux d'un collagène d'origine bovine.

La stabilité, l'aptitude à accélérer la régénération des tissus, l'aptitude à la résorption et la tolérance du collagène par l'organisme confèrent à cette macromolécule des propriétés de biocompatibilité qui en font un matériau support et vecteur de principe actif de choix pour des usages destinés à un contact intime et prolongé avec des tissus vivants blessés.

C'est pourquoi, la présente invention propose une composition pharmaceutique solide destinée à être introduite dans les poches parodontales pour leur traitement, qui se caractérise en ce qu'elle comprend, à titre de vecteur, du collagène, et à titre de principe actif au moins une substance antiseptique.

Les avantages de cette association sont très importants.

L'apport de collagène dans les lésions infra-osseuses est intéressant, puisque ce collagène offre une trame à la prolifération cellulaire et donc la possibilité de régénération des tissus après différenciation des cellules mésenchymateuses. L'adjonction d'un principe actif antibactérien peut assurer temporairement l'aseptie du site opératoire et réduire une dégradation trop rapide du matériau par le fait d'un afflux important de cellules de défense de l'organisme causé par l'inflammation. A titre de principe actif antibactérien, on peut utiliser différentes substances, telles que le métronidazole ou la chlorhexidine.

De préférence, selon la présente invention, la composition pharmaceutique comporte, à titre de principe actif, de la chlorhexidine ou un de ses sels, notamment choisis dans le groupe comportant le diacétate, le digluconate, le dichlorhydrate, car outre ses propriétés antibactériennes, ce principe actif présente des propriétés anti-inflammatoires.

La chlorhexidine est une molécule à caractère basique qui présente un spectre d'activité antibactérienne large, contre les bactéries gram$^+$ et gram$^-$.

In vivo, elle possède une bonne activité antiplaque qui persiste dans le temps. Cette activité rémanente est due aux propriétés d'adsorption de la chlorhexidine à l'hydroxyapatite de la dent et à ses interactions avec les composés salivaires comme les protéines et les mucines. Ainsi adsorbée, la chlorhexidine empêche la colonisation des surfaces dentaires et des muqueuses par les bactéries. Elle est progressivement relarguée dans le milieu où elle exerce encore une activité antibactérienne.

Par rapport aux antibiotiques, la chlorhexidine n'induit pas de résistance bactérienne, et ne conduit pas à des manifestations allergiques chez les patients. La toxicité de la chlorhexidine est très faible, ce principe actif n'étant pas absorbé dans le sang.

L'association de cette substance avec du collagène permet d'obtenir une activité thérapeutique locale bien déterminée, limitée dans l'espace au site d'application et modulable dans le temps.

Cette association est réalisée, de préférence avec un rapport en poids collagène/chlorhexidine, compris entre 1 et 3. La réalisation préférée de la composition selon l'invention fait intervenir un rapport en poids de collagène/chlorhexidine sensiblement égal à 2.

La forme galénique destinée à être introduite dans les poches parodontales pour assurer le contrôle de la plaque sous-gingivale ou pour combler les lésions parodontales infraosseuses doit avantageusement répondre à certains critères :
- la forme doit être stérile car elle est destinée à être implantée ;
- la forme doit être souple pour ne pas provoquer d'irritation, ni de gêne ;
- la forme doit être adaptable à différents volumes, car la taille et la configuration des poches sont variables, et
- la forme doit être de manipulation facile.

Différentes formes galéniques permettent de répondre à ces critères. De préférence, la composition selon la présente invention se présente sous forme de films ou de lyophilisats. Les films se présentent, de préférence, sous forme de petites feuilles, souples et résistantes d'une épaisseur moyenne d'environ 120 μm. Les lyophilisats se présentent, de préférence, sous forme de pastilles également souples et comprimables.

L'utilisation des films et des lyophilisats peut être associée à une intervention chirurgicale, la forme est alors appliquée par le praticien à l'endroit désiré. Les formes peuvent être utilisées après nettoyage des poches parodontales, sans chirurgie : elles sont alors introduites dans la lésion par le praticien à l'aide d'instruments appropriés. Elles ont pour rôle dans ce cas d'assurer un contrôle du développement de la plaque bactérienne sous-gingivale.

Ces films ou lyophilisats sont, le plus souvent, destinés à être utilisés sous forme d'implants ;

On définit ces formes comme des implants quelle que soit leur utilisation, c'est-à-dire associée ou non à une intervention chirurgicale, parce qu'elles se trouvent en contact prolongé avec des tissus vivants non soumis aux conditions extérieures lorsqu'ils sont dans un état sanitaire normal.

Quelle que soit sa forme galénique, la composition pharmaceutique selon la présente invention a une concentration en principe actif, par dose unitaire, comprise entre 0,5 mg et 5 mg. Cette concentration est, de préférence, d'environ 2,5 mg.

Le collagène utilisé dans la composition selon la présente invention est, de préférence, du collagène non déréticulé d'origine bovine.

La présente invention concerne aussi un procédé de préparation de ladite composition pharmaceutique, caractérisé en ce qu'il comporte les trois étapes suivantes :

a) on solubilise le collagène dans de l'acide acétique jusqu'à l'obtention d'une solution dont la concentration en collagène est comprise entre 0,5 g et 1,5 g pour 100 g de solution.

b) on solubilise le principe actif dans de l'eau distillée jusqu'à l'obtention d'une solution dont la concentration en principe actif est comprise entre 1 g et 2 g pour 100 g de solution.

c) on mélange entre elles les deux solutions obtenues à l'issue des étapes a) et b), puis on élimine les solvants.

Le mélange obtenu à l'issue de l'étape c) est par exemple coulé dans un récipient à fond plat pour permettre l'évaporation du solvant et l'obtention d'un film, ou bien réparti dans des flacons que l'on congèle et que l'on lyophilise.

La présente invention concerne, enfin, l'utilisation d'une association de collagène et d'un principe actif, tel que de la chlorhexidine ou un de ses sels, pour la fabrication d'une composition destinée au traitement d'affections parodontales par voie topique.

La réalisation technique des films et des lyophilisats sera mieux comprise à la lecture des exemples suivants donnés à simple titre d'illustration.

## 1. Généralités relatives à l'obtention de films et de lyophilisats

Ces deux formes peuvent être obtenues sous forme unitaire dont la quantité de principe actif est déterminée, ou sous forme "en vrac" présentant une répartition homogène du principe actif dont on utilise tout ou partie de la préparation suivant les besoins.

La concentration en principe actif de la forme galénique a été fixée après étude de l'activité antibactérienne de quantités variables de chlorhexidine présente dans des films, sur des bactéries isolées de la plaque dentaire.

L'inhibition de culture a été obtenue pour une concentration en chlorhexidine dans le film égale à environ 2,5 mg/cm$^2$.

Cette concentration a été jugée optimale dans la pratique et a été retenue tant pour les films que les lyophilisants. Mais il s'est avéré que, par exemple, la concentration de 0,5 mg/cm$^2$ est, dans certains cas, suffisamment active in vivo.

Par conséquence, les exemples de préparation des films et lyophilisats décrits ci-après sont adaptables à la réalisation de compositions dont la forme unitaire a une concentration en principe actif comprise entre 0,5 mg et 5 mg.

Le rapport collagène/chlorhexidine et fixé à 2/1 en poids. Les quantités ainsi mises en oeuvre permettent l'obtention de films et de lyophilisats souples et résistants.

## 2. Exemple de fabrication des films

Etape 1 : Solubilisation du collagène

Le collagène utilisé est un collagène natif non déréticulé d'origine bovine qui se présente sous forme de fibres. Les fibres sont dispersées et solubilisées sous agitation magnétique pendant 2 heures dans une solution d'acide acétique 0,1 M de telle manière que la concentration en collagène dans la solution soit égale à 1 % en poids.

Etape 2 : Addition du principe actif

La quantité de diacétate de chlorhexidine nécessaire pour obtenir un rapport de 2/1 en collagène/chlorhexidine est pesée.

Le diacétate de chlorhexidine est mis en solution dans une quantité d'eau distillée suffisante pour que la concentration de la solution soit égale à 1,5 % en poids.

La solution de diacétate de chlorhexidine et ajoutée à la solution de collagène sous agitation magnétique maintenue pendant 10 minutes environ.

Etape 3 : Obtention des films

Le mélange obtenu est coulé dans un récipient au fond parfaitement plat de surface déterminée, telle que la concentration en diacétate de chlorhexidine dans le produit fini soit égale à 2,5 mg/cm$^2$. Ainsi, par exemple, on peut utiliser, à titre de récipients, des boîtes de Pétri stériles dont la surface est d'environ 55,4 cm$^2$.

Si le mélange obtenu à l'issue de l'étape c) consiste en un volume global de 40 ml constitué par 30 ml d'une dispersion de 300 mg de films de collagène dans l'acide acétique 0,1 M, additionné de 10 ml d'eau distillée dans laquelle 150 mg d'acétate de chlorhexidine ont été solubilisés au préalable, alors le film obtenu présente une teneur en chlorhexidine de 2,7 mg/cm$^2$. Le récipient contenant le mélange est mis en étuve ventilée à une température inférieure ou égale à 30°C.

Le film est obtenu par évaporation du solvant, le temps nécessaire pour cette opération est de 2 à 3 jours.

Le film obtenu est pesé. Des carrés d'une surface égale à 1 cm$^2$ sont découpés dans le film, puis pesés et conditionnés individuellement dans des flacons de verre.

## 3. Exemple de fabrication des lyophilisats

La fabrication des lyophilisats est identique à la fabrication des films pour les étapes 1 et 2.

Etape 3 : Obtention des lyophilisats

Le mélange obtenu est réparti à la pipette dans des flacons à lyophilisation de 5 ml. Le volume de mélange prélevé doit correspondre à une quantité de chlorhexidine dans la forme finale égale à environ 2,5 mg. Ainsi, par exemple, on peut prélever un volume d'environ 0,72 ml du mélange obtenu à l'issue de l'étape c). Si ce mélange a la même composition que celle indiquée précédemment pour la préparation des films, alors les lyophilisats obtenus présentent une teneur en chlorhexidine de 2,7 mg par dose unitaire.

Les flacons sont soumis à la lyophilisation. La durée de l'opération est de 48 heures.

Elle consiste dans une première phase à la congélation du produit : durée 6 h 30.
- température de l'enceinte : - 50°C
- température au coeur du produit : - 45°C.

La deuxième phase est la lyophilisation : durée 24 h
- arrêt congélation sans réchauffage
- vide moyen qui assure un début d'évaporation du solvant
- puis vide poussé avec augmentation de la température jusque 35°C.

## 4. Stérilisation

L'opération finale de la fabrication des films et lyophilisats est la stérilisation.

La technique de stérilisation est par exemple la stérilisation à l'oxyde d'éthylène.

Elle est réalisée dans des conditions telles qu'il n'y ait pas altération des produits, c'est-à-dire :
- température 35°C-40°C
- humidité 60 %
- pression légèrement inférieure à la pression atmosphérique
- durée de l'opération 14 h
- composition du mélange gazeux : 50% d'air, 50% d'oxyde d'éthylène.

Après stérilisation, les flacons sont bouchés et sertis.

## 5. Contrôles

Les contrôles effectués sur les films et lyophilisats sont satisfaisants pour la stérilité et pour la teneur résiduelle en oxyde d'éthylène.

La libération de l'acétate de chlorhexidine in vitro a été étudiée pour les films et les lyophilisats.

Le produit pesé est retenu dans une cellule maintenue à la température de 37°C dans un bain marie thermostaté. Il est soumis au passage d'un liquide au débit de 0,5 ml/min. pendant 7 heures. Le liquide est une solution d'électrolytes dilués, simulant la salive, dont la composition est la suivante :

| | |
|---|---|
| - $CaCl_2$ | 0,6 mM |
| - $NaH_2PO_4,2H_2O$ | 1,8 mM |
| - $NaH\,CO_3$ | 12 mM |
| - HCl | 1 mM |
| pH : | 7,4. |

Le liquide est recueilli pour le dosage de l'acétate de chlorhexidine par la technique colorimétrique de Hoolbrook.

Les résultats sont présentés dans les tableaux suivants.

TABLEAUX DE RESULTATS

Acétate de chlorhexidine libéré exprimé en % de la quantité théorique d'acétate de chlorhexidine dans 7 lyophilisats

| Temps | lyophilisat | | | | | | | moyenne | écart-type |
|---|---|---|---|---|---|---|---|---|---|
| | 1 | 2 | 3 | 4 | 5 | 6 | 7 | | |
| 1 h | 26,75 | 47,5 | 40 | 45,4 | 41,4 | 53 | 34 | 41,15 | 8,09 |
| 2 h | 42,3 | 61,8 | 55 | 63 | 62 | 73,4 | 48,7 | 58 | 9,5 |
| 3 h | 50,4 | 68,5 | 64 | 71,8 | 71,3 | 82,7 | 55,5 | 66,3 | 10 |
| 4 h | 58,26 | 72,6 | 70 | 78,4 | 76,8 | 87 | 61,8 | 72,1 | 9,1 |
| 5 h | 63 | 74,3 | 74 | 81 | 80 | 89,8 | 66 | 75,4 | 8,5 |
| 6 h | 64,8 | 75,1 | 75,8 | 82 | 81 | 90,7 | 70 | 77 | 7,8 |
| 7 h | 65,8 | 76,3 | 77,2 | 82,7 | 82 | 91,5 | 73 | 78,3 | 7,5 |

Acétate de chlorhexidine libéré exprimé en % de la quantité théorique de chlorhexidine dans 4 films

| Temps | Film | | | | moyenne | écart-type |
|---|---|---|---|---|---|---|
| | 1 | 2 | 3 | 4 | | |
| 1 h | 48,4 | 46,3 | 50,9 | 67,7 | 53,3 | 8,45 |
| 2 h | 60,5 | 63,3 | 71,6 | 72,1 | 66,8 | 5,07 |
| 3 h | 67,37 | 68,1 | 76 | 74,4 | 71,4 | 3,78 |
| 4 h | 72,7 | 72,1 | 82 | 76 | 75,7 | 3,9 |
| 5 h | 77,8 | 75,5 | 87,5 | 77,1 | 79,4 | 4,7 |
| 6 h | 81 | 77,1 | 89,7 | 78 | 81,45 | 4,9 |

## 6. Essais cliniques

Les compositions selon l'invention ont démontré une excellente activité dans le traitement d'affections parodontales, tel que cela ressort notamment de l'étude clinique rapportée ci-après.

Cette étude clinique préliminaire chez l'homme a eu pour objectif de vérifier l'intérêt du système décrit pour le traitement des parodontites, c'est-à-dire :
- l'activité antibactérienne de la forme pharmaceutique due à la libération de la chlorhexidine
- la participation du collagène à la cicatrisation et à la restructuration du parodonte
- la résorbabilité du matériau
- la tolérance.
  Trois cas ont été étudiés :
- deux concernant l'utilisation du lyophilisat
- un concernant l'utilisation du film.
  Les trois cas sont des cas de parodontites complexes nécessitant une chirurgie par levée de lambeau.

Les patients sont soumis de la même manière à une préparation initiale qui consiste en un détartrage et surfaçage des lésions.

L'instauration d'un contrôle de plaque amenant l'indice d'Oleary en dessous de 10% et des bains de bouche avec une solution pure de digluconate de chlorhexidine à 0,2% pendant 2 minutes trois fois par jour.

Chaque cas est documenté par photographies, radiographies, sondage des poches et relevés des indices de plaque (PlI) et d'inflammation (GI) aux différents temps : 8 jours-15 jours-3 mois-6 mois.

EXEMPLE 1

**Cas n° 1 - 7 mois de recul**

1.1 - Traitement et résultats

Produit utilisé : lyophilisat de collagène-chlorhexidine
Patient : homme de 40 ans
Lésion traitée : dent 26, lésion interradiculaire de type III mésiale distale et palatine

Le niveau gingival marginal étant maintenu à la jonction émail-cément, les poches sont de 6 à 7 mm et la sonde Nabers pénètre la furcation à partir de toutes les faces. Après préparation initiale les indices PlI et GI sont de zéro. La mobilité dentaire est de III.

Un lambeau curetage est réalisé en regard de 26, les incisions sont intra-sulculaires sans éviction tissulaire. Le tissu de granulation est débridé, la lésion infra-osseuse est aménagée. Les surfaces radiculaires sont traitées mécaniquement et chimiquement avec une solution d'acide citrique à pH 1. Le lyophilisat de collagène-chlorhexidine est placé dans la zone la plus déclive de la lésion osseuse puis les lambeaux sont suturés de façon hermétique en toit au-dessus de la zone ainsi traitée. Un pansement chirurgical type Coë Pack est placé sur les faces vestibulaire et palatine. Des bains de bouche avec solution pure de digluconate de chlorhexidine à 0,2% sont prescrits à raison de 3 par jour pendant 2 minutes.
- A 8 jours, les sutures sont déposées, les tissus gingivaux, indemnes de toutes manifestations inflammatoires, sont parfaitement adaptés aux surfaces radiculaires et ne présentent pas de récession. Les indices de plaque et d'inflammation sont nuls. La mobilité est légèrement augmentée.
- A 15 jours, l'aspect inflammatoire des tissus gingivaux persiste, une légère récession gingivale est installée, la mobilité est de II$^+$, les indices sont maintenus.

Les contrôles réguliers jusqu'à 7 mois post-opératoire amènent l'observation d'une réduction totale de la mobilité et un maintien des résultats. Une récession importante met à jour la zone de la furcation mais chacune des 3 racines est parfaitement sertie par les tissus gingivaux sans que l'on puisse sonder plus de 2 mm, sans entraîner de saignement. Les soins de contrôle de plaque sont exécutés avec minutie.

1.2 - Discussion cas n° 1

Ce premier cas, à sept mois de recul, montre la parfaite tolérance du lyophilisat de collagène-chlorhexidine à court terme et à long terme.

Comparativement à des cas de ce type traités de façon classique, la cicatrisation a été accélérée, la récession "guidée" évitant le maintien de poches résiduelles.

Le collagène "in situ" relarguant la chlorhexidine semble avoir servi de support à une cicatrisation empêchant la fusée d'épithélium jusqu'au fond du défaut osseux. Ceci ne conjonction avec le traitement acide des surfaces radiculaires et soin apporté à l'herméticité des sutures.

Ce matériau, d'après la théorie des domaines de Melcher, interposé entre la surface radiculaire et l'intrados du lambeau a servi de protection évitant le contact direct des cellules du tissu conjonctif gingival avec la surface radiculaire rendue bio-compatible. Ceci peut donc avoir favorisé l'envahissement du site opératoire par les cellules en provenance du desmodonte aptes à réaliser, du moins sur une hauteur minime, l'installation d'une attache conjonctive d'après les travaux de Lindhe et Karring (1985).

## EXEMPLE 2

### Cas n° 2 - 6 mois de recul

2.1 - Traitement et résultats

Produit utilisé : lyophilisat de collagène-chlorhexidine
Patient : homme de 24 ans atteint de parodontite juvénile
Lésion traitée : dent 36 - lésion infra-osseuse interradiculaire
Le protocole est le même excepté l'adjonction classique lors de parodontite juvénile d'antibiothérapie (DOXYCLINE 4 jours, 10 jours arrêt 20 jours et reprise) ainsi que l'apport de matériau de comblement synthétique dans la lésion (2 doses de 1 mg), le lyophilisat de collagène-chlorhexidine est placé après l'insertion du matériau de comblement résorbable, dans la lésion interradiculaire côté lingual et les lambeaux sont collés par l'intrados au Tissucol (fibrinogène humain), puis suturés.
La poche dans ce site passe de 5 mm à 2 mm à 6 mois, la mobilité de III$^+$ est passée à I, aucune inflammation, ni dépôt de plaque ne sont relevés à 6 mois, le patient effectuant un parfait contrôle de plaque et demeurant sous inhibiteur de plaque.

2.2 Discussion cas n° 2

Le GT 2 est couplé à l'usage de "Synthograf" et de colle à la fibrine et semble potentialiser l'un et l'autre des produits.
Il est remarquable de noter que les lésions interradiculaires sont du plus mauvais pronostic en pratique classique et que ce cas comme le précédent a présenté une cicatrisation particulièrement favorable.

## EXEMPLE 3

### Cas n° 3 - 1 an de recul

3.1 Traitement

Produit utilisé : film de collagène-chlorhexidine dosé à 0,5 mg/cm$^2$
Patient : femme de 60 ans
Lésion traitée : dent 26 - poches infra-osseuses.
La lésion est le siège d'une intervention à lambeau "type lambeau esthétique d'accès" permettant le traitement des poches parodontales avec accès aux surfaces radiculaires et fermeture très hermétique du site opératoire.
La radiographie conjuguée au sondage des poches mettent en évidence la présence de poches infra-osseuses de 8 mm en mésial, 5 mm en vestibulaire, 4 mm en distal, le sondage palatin est de 3 mm.
Le Pbi et le SBi ont une valeur zéro, la mobilité dentaire est de III (plus de 1 mm de part et d'autre de l'axe médian dans un sens vestibulo-palatin).
L'intervention a constitué, après levée d'un lambeau muco-périosté vestibulaire et palatin en un curetage des tissus de granulation et une préparation radiculaire mécanique à la curette ainsi que d'une préparation chimique des surfaces cémentaires par application d'une solution d'acide citrique saturé à pH 1.
Un soin particulier est mis au débridement de la lésion infra-osseuse mésio-vestibulaire qui confirme l'existence d'une lésion interradiculaire mésiale de type II, la sonde pénétrant de 2 mm la furcation à partir de la face mésiale. Le film collagène-chlorhexidine est appliqué d'une part sur la surface radiculaire vestibulaire traitée, à l'intrados du lambeau, et d'autre part un fragment en est inséré dans la lésion interradiculaire.

Après réapplication des lambeaux et coaptation intime des berges vestibulaire et palatine maintenue par des sutures interproximales par point de matelassier verticaux, un pansement chirurgical type Coë Pack est mis en place pour 8 jours et des bains de bouche d'une solution de dig1 conate de chlorhexidine à 0,2% sont prescrits à raison de 3 bains de bouche par jour pendant 2 minutes.

3.2 - Résultats

- 8 jours après l'intervention le pansement est déposé, le "film collagène-chlorhexidine" est apparent dans la région cervicale, la fibro-muqueuse n'est pas adaptée hermétiquement à la racine dans les zones vestibulaires et mésiales.

Aucun signe inflammatoire n'est décelé, il n'y a pas de dépôt de plaque bactérienne au balayage par la sonde. Il n'y a pas d'halitose. La mobilisation dentaire est augmentée.

La patiente ne mentionne ni douleur, ni gêne.

Les sutures sont déposées, aucun pansement n'est remis et la reprise de manoeuvres d'hygiène méticuleuses est prescrite ainsi que la poursuite des bains de bouche à la chlorhexidine.

- 15 jours après l'intervention le film a disparu de la lésion soit par résorption, soit par élimination mécanique. On observe une cicatrisation en architecture négative au niveau de l'insertion du film. Le site est légèrement inflammatoire et douloureux avec développement de plaque dentaire.

- 3 mois aprés l'intervention, l'architecture négative a disparu, excepté dans la région mésiale. Le lambeau vestibulaire est parfaitement réappliqué mais son bord marginal a subi une récession de 3 mm réduisant la profondeur de poche à 2 mm.

La profondeur de poche en position mésiale accédant à la zone de furcation est réduite de 8 mm à 6 mm. La mobilité est réduite à II selon l'échelle de l'ARPA.

- A 1 an, la mobilité de 26 est physiologique.

La profondeur de poche vestibulaire est de 2 mm, elle se maintient à 4 mm en mésial avec un défaut d'architecture gingivale de la papille. Les indices de plaque (PlI) et d'inflammation (GI) sont de zéro.

Ces études cliniques préliminaires révèlent la parfaite tolérance des lyophilisats de collagène-chlorhexidine, utilisés pour le comblement des poches parodontales. Le collagène améliore la cicatrisation des lésions traitées et semble participer à l'instauration de nouvelles structures tissulaires.

La libération de chlorhexidine au site d'implantation empêche le développement de plaque et maintient l'aseptie de la lésion après l'opération.

Les films de collagène-chlorhexidine sont moins intéressants en application au niveau des lésions infra-osseuses nécessitant chirurgie.

Ils pourront être réservés pour une application dans la poche parodontale ne nécessitant pas de chirurgie, où ils assureront le contrôle du développement de la plaque dentaire par une libération de chlorhexidine qui sera maintenue au moins 8 heures, compte tenu des résultats de libération obtenus in vitro.

**Revendications**

**Revendications pour les Etats contractants suivants : BE, CH, FR, GB, IT, LI, LU, NL, SE**

1. Composition pharmaceutique solide, destinée à être introduite dans les poches parodontales pour leur traitement, caractérisée en ce qu'elle comprend, à titre de vecteur, du collagène et, à titre de principe actif, au moins une substance antiseptique choisie parmi le métronidazole, la chlorhexidine ou l'un de ses sels, notamment le diacétate, le digluconate et le dichlorhydrate.

2. Composition pharmaceutique selon la revendication 1, caractérisée en ce que le rapport en poids collagène-chlorhexidine est compris entre 1 et 3.

3. Composition pharmaceutique selon l'une des revendications 1 et 2, caractérisée en ce qu'elle se présente sous forme de film.

4. Composition pharmaceutique selon l'une des revendications 1 à 3, caractérisée en ce qu'elle se présente sous forme de lyophilisat.

5. Composition pharmaceutique selon l'une des revendications 1 à 4, caractérisée en ce qu'elle se présente sous forme d'un implant.

**6.** Composition pharmaceutique selon l'une des revendications 1 à 5, caractérisée en ce que sa concentration en principe actif par dose unitaire est comprise entre 0,5 mg et 5 mg.

**7.** Composition pharmaceutique selon l'une des revendications 1 à 6, caractérisée en ce que le collagène utilisé est un collagène natif non déréticulé d'origine bovine.

**8.** Procédé de préparation d'une composition pharmaceutique selon l'une des revendications 1 à 7, caractérisé en ce qu'il comporte les étapes suivantes :
a) on solubilise le collagène dans de l'acide acétique jusqu'à l'obtention d'une solution dont la concentration en collagène est comprise entre 0,5 et 1,5 g pour 100 g de solution,
b) on solubilise le principe actif dans de l'eau distillée jusqu'à l'obtention d'une solution dont la concentration en principe actif est comprise entre 1 g et 2 g pour 100 g de solution,
c) on mélange entre elles les deux solutions obtenues à l'issue des étapes a) et b), puis on élimine les solvants.

**9.** Utilisation d'une association de collagène et d'un principe actif, choisi parmi le métronidazole et la chlorhexidine ou un de ses sels, pour la fabrication d'une composition destinée au traitement d'affections parodontales par voie topique.

**Revendications pour les Etats contractants suivants : AT, ES, GR**

**1.** Procédé de préparation d'une composition pharmaceutique solide, destinée à être introduite dans les poches parodontales pour leur traitement, caractérisée en ce qu'elle comprend, à titre de vecteur, du collagène et, à titre de principe actif, au moins une substance antiseptique choisie parmi le métronidazole, la chlorhexidine ou l'un de ses sels, notamment le diacétate, le digluconate et le dichlorhydrate, caractérisé en ce qu'il comporte les étapes suivantes :
a) on solubilise le collagène dans de l'acide acétique jusqu'à l'obtention d'une solution dont la concentration en collagène est comprise entre 0,5 et 1,5 g pour 100 g de solution,
b) on solubilise le principe actif dans de l'eau distillée jusqu'à l'obtention d'une solution dont la concentration en principe actif est comprise entre 1g et 2 g pour 100 g de solution,
c) on mélange entre elles les deux solutions obtenues à l'issue des étapes a) et b), puis on élimine les solvants.

**2.** Procédé selon la revendication 1, caractérisé en ce que le rapport en poids collagène-chlorhexidine est compris entre 1 et 3.

**3.** Procédé selon l'une des revendications 1 et 2, caractérisé en ce que ladite composition se présente sous forme de film.

**4.** Procédé selon l'une des revendications 1 à 3, caractérisé en ce que ladite composition se présente sous forme de lyophilisat.

**5.** Procédé selon l'une des revendications 1 à 4, caractérisé en ce que ladite composition se présente sous forme d'un implant.

**6.** Procédé selon l'une des revendications 1 à 5, caractérisé en ce que la concentration en principe actif par dose unitaire est comprise entre 0,5 mg et 5 mg.

**7.** Procédé selon l'une des revendications 1 à 6, caractérisé en ce que le collagène utilisé est un collagène natif non déréticulé d'origine bovine.

**Claims**
**Claims for the following Contracting States : BE, CH, FR, GB, IT, LI, LU, NL, SE**

**1.** A solid pharmaceutical composition for introduction into periodontal pockets in order to treat them, which comprises collagen as the vehicle and at least one antiseptic substance as the active principle, chosen among metronidazole, chlorhexidine or one of its salts, in particular the diacetate, the digluconate and the dihydrochloride.

11

EP 0 233 429 B1

**2.** A pharmaceutical composition as claimed in claim 1, wherein the weight ratio collagen/chlorhexidine is between 1 and 3.

**3.** A pharmaceutical composition as claimed in claim 1 and 3, which is in the form of a film.

**4.** A pharmaceutical composition as claimed in one of claims 1 to 3, which is in the form of a lyophilizate.

**5.** A pharmaceutical composition as claimed in one of claims 1 to 4, which is in the form of an implant.

**6.** A pharmaceutical composition as claimed in one of claims 1 to 5, whose active principle concentration is between 0,5 mg and 5 mg per unit dose.

**7.** A pharmaceutical composition as claimed in one of claims 1 to 6, wherein the collagen used is a non-decrosslinked native collagen of bovine origin.

**8.** A process for the preparation of a pharmaceutical composition as claimed in one of claims 1 to 7, which comprises the following steps:
a) the collagen is solubilized in acetic acid until a solution with a collagen concentration of between 0,5 g and 1,5 g per 100 g of solution is obtained;
b) the active principle is solubilized in distilled water untill a solution with an active principle concentration of between 1 g and 2 g per 100 g of solution is obtained; and
c) the two solutions obtained after steps a) and b) are mixed together and the solvents are then removed.

**9.** Use of an association of collagen with an active principle, chosen among metronidazole and chlorhexidine or one of its salts, for the production of a composition for the topical treatment of periodontal complaints.

**Claims for the following Contracting States : AT, ES, GR**

**1.** A process for the preparation of a solid pharmaceutical composition for introduction into periodontal pockets in order to treat them, which comprises collagen as the vehicle and at least one antiseptic substance as the active principle, chosen among metronidazole, chlorhexidine or one of its salts, in particular the diacetate, the digluconate and the dihydrochloride, which comprises the following steps:
a) the collagen is solubilized in acetic acid until a solution with a collagen concentration of between 0,5 g and 1,5 g per 100 g of solution is obtained;
b) the active principle is solubilized in distilled water untill a solution with an active principle concentration of between 1 g and 2 g per 100 g of solution is obtained; and
c) the two solutions obtained after steps a) and b) are mixed together and the solvents are then removed.

**2.** A process as claimed in claim 1, wherein the weight ratio collagen, chlorexidine is between 1 and 3.

**3.** A process as claimed in one of claims 1 and 2 in which said composition is in the form of a film.

**4.** A composition as claimed in one of claims 1 to 3, in which said composition is in the form of a lyophilizate.

**5.** A process as claimed in one of claims 1 to 4 in which said composition is in the form of an implant.

**6.** A pharmaceutical composition as claimed in one of claims 1 to 5, in which active principle concentration is between 0,5 mg and 5 mg per unit dose.

**7.** A process as claimed in one of claims 1 to 6, wherein the collagen used is a non decrosslinked native collagen of bovine origin.

12

**Patentansprüche**
**Patentansprüche für folgende Vertragsstaaten : BE, CH, FR, GB, IT, LI, LU, NL, SE**

1. Feste pharmazeutische Zusammensetzung, die in die Parodontaltaschen eingeführt werden soll, um sie zu behandeln, dadurch gekennzeichnet, daß sie umfaßt bzw. enthält Kollagen als Träger und mindestens eine antiseptische Substanz, ausgewählt aus Metronidazol, Chlorhexidin oder seinen Salzen, insbesondere seinem Diacetat, Digluconat und Dihydrochlorid, als aktives Prinzip (Wirkstoff)

2. Pharmazeutische Zusammensetzung nach Anspruch 1, dadurch gekennzeichnet, daß das Kollagen/Chlorhexidin-Gewichtsverhältnis zwischen 1 und 3 liegt.

3. Pharmazeutische Zusammensetzung nach einem der Ansprüche 1 und 2, dadurch gekennzeichnet, daß sie in Form eines Films vorliegt.

4. Pharmazeutische Zusammensetzung nach einem der Ansprüche 1 bis 3, dadurch gekennzeichnet, daß sie in Form eines Lyophilisats vorliegt.

5. Pharmazeutische Zusammensetzung nach einem der Ansprüche 1 bis 4, dadurch gekennzeichnet, daß sie in Form eines Implantats vorliegt.

6. Pharmazeutische Zusammensetzung nach einem der Ansprüche 1 bis 5, dadurch gekennzeichnet, daß ihre Konzentration an aktivem Prinzip (Wirkstoff) pro Einheitsdosis zwischen 0,5 und 5 mg liegt.

7. Pharmazeutische Zusammensetzung nach einem der Ansprüche 1 bis 6, dadurch gekennzeichnet, daß es sich bei dem verwendeten Kollagen um ein vom Rind stammendes nichtderetikuliertes natives Kollagen handelt.

8. Verfahren zur Herstellung einer pharmazeutischen Zusammensetzung nach einem der Ansprüche 1 bis 7, dadurch gekennzeichnet, daß es die folgenden Stufen umfaßt:
   a) man löst das Kollagen in Essigsäure bis zur Erzielung einer Lösung, deren Kollagenkonzentration zwischen 0,5 und 1,5 g auf 100 g Lösung liegt,
   b) man löst das aktive Prinzip (den Wirkstoff) in destilliertem Wasser bis zur Erzielung einer Lösung, deren Konzentration an aktivem Prinzip (Wirkstoff) zwischen 1 und 2 g auf 100 g Lösung liegt, und
   c) man mischt die in den Stufen (a) und (b) erhaltenen beiden Lösungen miteinander, dann eliminiert man die Lösungsmittel.

9. Verwendung einer Assoziation von Kollagen mit einem aktiven Prinzip (Wirkstoff), ausgewählt aus Metronidazol und Chlorhexidin oder seinen Salzen, für die Herstellung einer Zusammensetzung für die topische Behandlung von Parodontalerkrankungen.

**Patentansprüche für folgende Vertragsstaaten : AT, ES, GR**

1. Verfahren zur Herstellung einer festen pharmazeutischen Zusammensetzung, die in die Parodontaltaschen eingeführt werden soll, um diese zu behandeln, dadurch gekennzeichnet, daß sie umfaßt bzw. enthält Kollagen als Träger und mindestens eine antiseptische Substanz, ausgewählt aus Metronidazol, Chlorhexidin oder einem seiner Salze, insbesondere seinem Diacetat, Digluconat und Dihydrochlorid, als aktives Prinzip (Wirkstoff), dadurch gekennzeichnet, daß es die folgenden Stufen umfaßt:
   a) man löst das Kollagen in Essigsäure bis zur Erzielung einer Lösung, deren Kollagenkonzentration zwischen 0,5 und 1,5 g auf 100 g Lösung liegt,
   b) man löst das aktive Prinzip (den Wirkstoff) in destilliertem Wasser bis zur Erzielung einer Lösung, deren Konzentration an aktivem Prinzip (Wirkstoff) zwischen 1 und 2 g auf 100 g Lösung liegt, und
   c) man mischt die in den Stufen (a) und (b) erhaltenen beiden Lösungen miteinander, dann eliminiert man die Lösungsmittel.

2. Verfahren nach Anspruch 1, dadurch gekennzeichnet, daß das Kollagen/Chlorhexidin-Gewichtsverhältnis zwischen 1 und 3 liegt.

3. Verfahren nach einem der Ansprüche 1 und 2, dadurch gekennzeichnet, daß die genannte Zusammensetzung in Form eines Films vorliegt.

4. Verfahren nach einem der Ansprüche 1 bis 3, dadurch gekennzeichnet, daß die genannte Zusammensetzung in Form eines Lyophilisats vorliegt.

5. Verfahren nach einem der Ansprüche 1 bis 4, dadurch gekennzeichnet, daß die genannte Zusammensetzung in Form eines Implantats vorliegt.

6. Verfahren nach einem der Ansprüche 1 bis 5, dadurch gekennzeichnet, daß die Konzentration an aktivem Prinzip (Wirkstoff) pro Einheitsdosis zwischen 0,5 und 5 mg liegt.

7. Verfahren nach einem der Ansprüche 1 bis 6, dadurch gekennzeichnet, daß das verwendete Kollagen ein vom Rind stammendes nicht-deretikuliertes natives Kollagen ist.